Europäisches Patentamt

European Patent Office

Office européen des brevets

(11) Veröffentlichungsnummer: **0 226 182 B1**

(12) **EUROPÄISCHE PATENTSCHRIFT**

(45) Veröffentlichungstag der Patentschrift: **24.04.91**

(51) Int. Cl.5: **G01N 33/544**, G01N 33/52, //G01N33/573,G01N33/78

(21) Anmeldenummer: **86117262.5**

(22) Anmeldetag: **11.12.86**

(54) **Reagenzpapier für die immunologische Analyse und Verfahren zu seiner Herstellung.**

(30) Priorität: **11.12.85 DE 3543749**

(43) Veröffentlichungstag der Anmeldung:
**24.06.87 Patentblatt 87/26**

(45) Bekanntmachung des Hinweises auf die
Patenterteilung:
**24.04.91 Patentblatt 91/17**

(84) Benannte Vertragsstaaten:
**AT BE CH DE ES FR GB GR IT LI LU NL SE**

(56) Entgegenhaltungen:
**EP-A- 0 082 345**
**EP-A- 0 097 952**
**EP-A- 0 162 302**
**GB-A- 1 369 139**

JOURNAL OF IMMUNOLOGICAL METHODS,
Band 83, 24. Oktober 1985, Seiten 55-60, Elsevier Science Publishers B.V., Amsterdam,
NL; M. SARKAR et al.: "Immobilization of
antibodies on a new solid phase for use in
ELISA"

(73) Patentinhaber: **BOEHRINGER MANNHEIM GMBH** .

Patentabteilung, Abt. E Sandhofer Strasse
112-132 Postfach 31 01 20
W-6800 Mannheim 31 Waldhof(DE)

(72) Erfinder: **Baier, Manfred, Dr.rer.nat.**
**Tiefentalweg 10**
**W-8124 Seeshaupt(DE)**
Erfinder: **Kaspar, Klaus Peter, Dr.rer.nat.**
**Calle Fulgencio R. Moreno 5309**
**Asuncion(PY)**
Erfinder: **Schäfer, Rainer, Dr.rer.nat.**
**Tiefentalweg 29**
**W-8124 Seeshaupt(DE)**
Erfinder: **Träger, Ulrich, Dr.rer.nat.**
**Eschkopfstr. 6**
**W-6703 Limburgerhof(DE)**
Erfinder: **Nötzel, Siegfried, Dr.rer.nat.**
**Silberg. 38**
**W-6901 Wilhelmsfeld(DE)**

(74) Vertreter: **Huber, Bernhard, Dipl.-Chem. et al**
**Patentanwälte H. Weickmann, Dr. K. Fincke**
**F.A. Weickmann, B. Huber Dr. H. Liska, Dr. J.**
**Prechtel Möhlstrasse 22 Postfach 860 820**
**W-8000 München 86(DE)**

EP 0 226 182 B1

**Beschreibung**

Die Erfindung betrifft ein Reagenzpapier für die immunologische Analyse, ein Verfahren zu seiner Herstellung und seine Verwendung.

Es ist bekannt, Antikörper und Antigene durch heterogene immunologische Analyse (Immunoassays) zu bestimmen, wobei man im Prinzip nach den folgenden zwei Methoden arbeitet:

Zur Bestimmung spezifischer Antikörper, insbesondere zur Bestimmung von Antiallergenen, wird ein für den zu bestimmenden Antikörper spezifisches Antigen auf einem wasserunlöslichen Trägermaterial oder Immunadsorbens fixiert (immobilisiert); der so behandelte Reagenzträger wird dann mit dem auf Antikörper zu untersuchenden Serum in Kontakt gebracht, wobei die für das auf dem Reagenzträger fixierte Antigen spezifischen Antikörper mit dem Antigen in einer immunologischen Reaktion kombinieren. Nach dem Waschen wird dann in einem zweiten Schritt der Träger mit Antikörpern in Kontakt gebracht, die spezifisch gegen die zu bestimmenden Antikörper oder Immunglobuline sind; diese Antikörper sind markiert, meistens entweder radioaktiv oder mit einem Enzym. Nach dem Waschen wird dann die Markierung, z. B. die Radioaktivität (sog. radioimmunologischer Test), oder die enzymatische Aktivität (sog. enzymimmunologischer Test), z. B. mittels colorimetrischer Analyse, bestimmt.

Nach einer zweiten Methode, der sog. "Sandwich-Methode", insbesondere zur Bestimmung bestimmter Serumglobuline, die als Antigene fungieren, wird an dem Trägermaterial ein gegen die zu bestimmenden Antigene spezifisch wirkender Antikörper fixiert. Dieser Träger wird dann in Kontakt mit dem die zu bestimmenden Antigene enthaltenden Serum gebracht, wobei die Antigene mit den Antikörpern kombinieren. Nach dem Waschen wird der Träger mit Antikörpern, die markiert sind und gegen dasselbe Antigen gerichtet sind, in Kontakt gebracht, und wie bei der ersten Methode die auf dem Träger verbleibende Markierung, z. B. die Radioaktivität oder enzymatische Aktivität bestimmt.

Aus der EP-A 0 097 952 sind mehrschichtige Analyse-Elemente zur fluorimetrischen Bestimmung von Analyten bekannt, die aus porösen faserhaltigen Blättern aufgebaut sind und die in einem Blatt eine vorbestimmte Menge an Protein enthalten. Das Protein in diesen Analyse-Ele-menten ist durch Absorption oder durch sogenannte "Linker" kovalent gebunden.

Zur Fixierung von Proteinen (Antikörpern oder Antigenen) werden als Trägermaterialien Kunststoffe, wie Polystyrol, Vinylpolymere, Polypropylen, Polycarbonat, Silikone, Gummi oder behandeltes Glas verwendet (vgl. z. B. E. T. Maggio, "Enzyme Immuno Assay" GAG Press, Florida, 1980, insbesondere Seiten 175 bis 178), oder Polysaccharide, wie z. B. Zellulose (vgl. z. B. EP-A-063 064; Bioengineering 16 (1974) 997-1003; C. J. Sanderson und D. V. Wilson, Immunology 20 (1971) 1061-1065)

Ein Nachteil der Fixierung von Proteinen auf Kunst-stoffoberflächen ist es, daß der Antikörper an die Trägeroberfläche adsorptiv nicht ausreichend fest gebunden wird, wodurch beim Waschen und bei der-Inkubation große Mengen an Antikörper wieder desorbiert werden, was eine Verringerung der Präzision und Sensitivität zur Folge hat; einen weiteren Nachteil stellt auch die begrenzte Adsorptionskapazität von Kunststoffoberflächen dar (vgl. z. B. E. T. Maggio, 1. c. S. 175). Um eine bessere Fixierung der Antikörper durch eine kovalente Bindung zu erreichen, wurde auch versucht, die Kunststoffoberflächen zu aktivieren. So ist es z. B. bekannt, Polypropylen-Röhren mit Glutaraldehyd vorzubehandeln (vgl. z. B. S. Avrameas et al, "Immuno Enzymatic Techniques" Elsevier Science Publishers, S. 163; G. H. Parsons, jr., in "Methods in Enzymology", Vol. 73, Immunochemical Techniques, Part B, Academic Press 1981, Seite 224 - 239, insbesondere Seite 234).

Ein Verfahren zur Fixierung von Proteinen auf Polysaccharid-Träger ist z. B. aus der EP-A-063 064 bekannt; danach werden die Polysaccharide, z. B. Zellulose oder Zellulosederivate, durch Oxidation mit Natriumperjodat aktiviert und nach Kupplung mit dem Antigen oder Antikörper wird der Reagenzträger mit Natriumborhydrid reduziert; als Kupplungszeiten werden 8 bis 15 Stunden angegeben. Nach anderen Methoden werden Polysaccharide wie Zellulose oder Sephadex ®-Teilchen durch Behandlung mit Bromcyan aktiviert (vgl. z. B. L. Wide in "Methods in Enzymology", 73, 1. c., Seite 203-224).

Ein Nachteil dieser Verfahren sind geringe Beladungsdichte der Träger und lange Kopplungszeiten; bei den Kunststoffträgern kommt hierzu noch die beim Inkubationsschritt während der immunologischen Bestimmung erfolgende Desorption, sowie die Schwierigkeit, Vliese (flächige, schneidbare Reagenzträger) herzustellen; bei den Polysacchariden die Umständlichkeit der Verfahren (zusätzliche Behandlung mit Bromcyan oder Perjodat, wobei die Perjodatbehandlung noch einen weiteren Reduktionsschritt erforderlich macht), die Gefährlichkeit von Bromcyan, und die Ausbildung unspezifischer Bindungen, durch die der Leerwert erhöht und das Meßergebnis verfälscht wird. Außerdem ist die ausgebildete kovalente Bindung nicht stabil und führt zur teilweisen Ablösung des Proteins, wodurch eine Verringerung der Empfindlichkeit beobachtet wird.

Eine weitere Fixierungsmethode für ein sandwichgängiges Immunosorbens ist die Präzipitatbildung

2

(Immunpräzipitate; vgl. z. B. PGT-WO 82/02601 und US-A-3,888,629). Diese Fixierungsmethode besitzt jedoch den Nachteil, daß man einen zusätzlichen Anti-Antikörper benötigt oder für jeden Test ein individueller Reagenzträger optimiert werden muß.

Eine Aufgabe der vorliegenden Erfindung ist deshalb die Bereitstellung eines Reagenzpapieres für die immunologische Analyse, das die vorstehend genannten Nachteile nicht aufweist und eines Verfahrens zu seiner Herstellung, mit dem auf einfache, rasche, wirtschaftliche und kontinuierliche Weise auch im technischen Maßstab Reagenzien für die immunologische Analyse ohne Präzipitatbildung mit homogener Verteilung, hoher Bindekapazität, geringer unspezifischer Adsorption und hoher Stabilität gebunden werden können. Eine weitere Aufgabe der Erfindung ist die Schaffung eines Reagenzpapieres der angegebenen Art mit gesteigerter Bindekapazität ohne Steigerung der unspezifischen Adsorption.

Diese Aufgabe wird gelöst durch ein Reagenzpapier, gekennzeichnet durch ein Faservlies aus einer Mischung eines Zellulose-Bestandteiles und einer Synthesfaser, worin das Gewichtsverhältnis von Zellulose-Bestandteil Synthesefaser von 1 bis 90 zu 99 nis 10 beträgt, wobei das Faservlies mit Perjodat aktiviert und mit einem sauer vorbehandelten Protein beladen ist.

Gegenstand der Erfindung ist auch ein Verfahren zur Herstellung eines solchen Reagenzpapieres, wobei man ein Faservlies, das aus einer Mischung von einem Zellulosebestandteil und einer Synthesefaser besteht, in der bezogen auf das Gewicht das Mischungsverhältnis von Zellulosebestandteil zu Synthesefaser 1 bis 90 zu 99 bis 10 beträgt, durch eine Behandlung mit Perjodat aktiviert und das so aktivierte Faservlies mit einem sauer behandelten Protein belädt und ungebundenes Protein entfernt.

Ein weiterer Gegenstand der Erfindung ist auch die Verwendung eines solchen Reagenzpapieres als Reagenzträger für die heterogene immunologische Analyse. Es wurde nämlich gefunden, daß bereits eine Zumischung von geringen Mengen Zellulosefasern zu Synthesefasern bzw. Synthesefasern zu Zellulosefasern ausreicht, um eine Verbesserung des Reagenzträgers hinsichtlich der Verwendung bei der immunologischen Analyse zu erreichen. Eine deutliche Verbesserung wurde erhalten, wenn 1 % oder mehr Zellulosefasern bzw. 10 % oder mehr Synthesefasern zugemischt wurden. Das jeweilige Optimum ist insbesondere abhängig von den in der Analyse verwendeten Reagenzien, der Art der Fasern und vom Testverfahren. Das Optimum kann durch wenige Versuche (z. B. Messung der Bindekapazität für Antikörper, Messung der unspezifischen Bindung an das Vlies) ermittelt werden.

Das Gewichtsverhältnis Zellulose-Bestandteil/Synthesefasern beträgt vorzugsweise 10 bis 80/20 bis 90, und insbesondere 20 bis 40/60 bis 80. Als Zellulosebestandteil eignet sich jede Zellulosefaser, wie z. B. Zellulose in Form von Baumwollfasern, Fasern aus gebleichtem Sulfitzellstoff oder Sulfatzellstoff, oder Baumwoll-Linters (Linters). Es können auch zwei oder mehrere solcher Fasern im Gemisch verwendet werden.

Synthesefasern sind vorzugsweise Fasern aus Polyamid, Zellwolle oder Glasfasern, und insbesondere Polyesterfasern. Weitere geeignete Synthesefasern sind z. B. Polyacrylnitril-, Polyethylen- oder Polypropylenfasern. Auch hier können Gemische aus zwei oder mehreren Synthesefasern verwendet werden. Für die Vliesstruktur kann ein Zusatz von Bindefasern zweckmäßig sein, wie z. B. der Zusatz von Polyvinylalkohol-, Polyurethan-, Polystyrol-und/oder Polyvinylchlorid-Fasern, vorzugsweise in einer Menge von 0,1 bis 30 Gew.-%, bezogen auf die Menge an Zellulose-Bestandteil. Ebenso kann die Vliesstruktur durch Zusatz von Naßfestmitteln wie Harnstoff-Formaldehyde, Melamin-Formaldehyde oder Polyamide-Epichlorhydrine in einer Menge von 0,1 bis 30 Gew.-%, bezogen auf die Menge an Zellulose-Bestandteil verbessert werden. Beispielsweise kann Etadurin ® (Hersteller: Akzo Chemie), ein Polyamidaminepichlor-Hydrinharz, verwendet werden. Eine besonders bevorzugte Vlieszusammensetzung ist z. B. eine solche aus Polyester und Zellulose im Verhältnis 6:4 bis 8:2, und insbesondere aus 80 Gew.-% Polyester und 20 Gew.-% Sulfitzellulose. Die Eigenschaften der für das Faservlies verwendeten Fasern entsprechen in der Regel den für derartige Fasern üblichen Eigenschaften, wie z. B. Faserlänge, Faserdicke und Reinheit.

Bevorzugt werden erfindungsgemäß Faservliese verwendet, die nach dem Verfahren der Erfindung hergestellt sind.

Die Perjodat-Behandlung kann unter den für eine solche Behandlung aus dem Stand der Technik zur Fixierung von Proteinen auf Zellulose-Träger bekannten Bedingungen erfolgen. Dabei ist es möglich, die Fasern, insbesondere nur die Zellulosefasern, vor der Bildung des Faservlieses mit Perjodat zu behandeln, z. B. eine 1%ige Fasersuspension in wäßriger Natriumperjodat-Lösung; vorzugsweise erfolgt jedoch eine Behandlung des fertigen Faservlieses (Papier), wodurch in der Regel eine bessere Homogenität des fertigen Reagenzpapieres erhalten wird. Die Aktivierung des Vliesmaterials kann zweckmäßigerweise z. B. in einem Baumfärbeapparat erfolgen, wobei 10 bis 50 m² große Vliesflächen in einem Ansatz oxidiert werden können. Als Oxidationsmedium wird vorzugsweise eine 5 bis 15, insbesondere 10 millimolare wäßrige $NaJO_4$-Lösung verwendet, wobei die Perjodatmenge zweckmäßigerweise bei 1 bis 5 mMol, insbesondere bei ca. 2,5 mMol/g Vlies liegt. Die Behandlungsdauer (Oxidationszeit) kann eine bis mehrere Stunden,

EP 0 226 182 B1

vorzugsweise ca. zwei Stunden, betragen. Nach der Oxidation wird das Vliesmaterial perjodatfrei gewaschen, was z. B. im Baumfärbeapparat erfolgen kann, und anschließend getrocknet.

Die Herstellung des Faservlieses (Papier) kann auf eine der zur Vliesherstellung üblichen Arten erfolgen, z. B. indem man eine Faserstoffsuspension aus Zellstoffpulpe und Synthesefasern mit hoher Verdünnung auf ein Papiermaschinensieb aufbringt, absaugt und anschließend trocknet. Das Vlies (Papier) besitzt vorteilhafterweise ein Flächengewicht von 100 bis 250 g/m², und weist eine große Offenporigkeit auf. Die Dicke beträgt vorzugsweise 0,1 bis 1,5 mm, insbesondere 0,7 bis 1,0 mm.

Die Beladung des mit Perjodat behandelten, oxidierten Vliesmaterials mit dem sauer vorbehandelten Protein kann durch Imprägnieren des Faservlieses mit einer Lösung des Proteins erfolgen, z. B. in einer für solche Zwecke üblichen Imprägnieranlage. Das imprägnierte Vliesmaterial wird dann getrocknet, gegebenenfalls in einer Nachbehandlung zur Entfernung von ungebundenem Protein gewaschen, z. B. mit wäßrigen Pufferlösungen vom pH = 6 bis 8, und anschließend getrocknet.

Die Änderung der physikalisch-chemischen Eigenschaften von Antikörpern durch Variation von pH-Wert, Ionenstärke und Temperatur ist bekannt. So beschreiben z. B. Vandenbrand et al., Molecular Immonology, 18 Nr. 7 (1982) 621-631, eine Zunahme der Hydrophobizität (die sich in einer verstärkten Wechselwirkung mit Lipid-Modell-Membranen zeigt), wenn die Antikörper zunächst gegen einen Puffer vom pH = 2 und dann gegen einen Puffer vom pH = 7 dialysiert werden. J. J. Gonvers et al, BBA 251 (1971) 262-273 beschreiben, daß Kaninchen-IgG nach Inkubation bei pH < 4,0 Oligomere/Dimere bildet. L. W. Hoyer et al, Immunochemistry, 5 (1968) 277-292 beschreiben, daß bei der Antikörper-Desorption von Immunadsorbern im Bereich vom pH = 5 bis 7 ein Salzzusatz die Elution fördert, während im pH-Bereich von 1 bis 3 die Elution durch Salzzusatz verringert wird. Es wird daraus auf hydrophobe Wechselwirkungen bei niedrigen pH-Werten geschlossen. Aus der JP-OS-57-74 663 sowie aus E. Ishikawa et al, Journal of Immunoassay 1 (3) (1980) 385-398 ist es bekannt, eine saure Vorbehandlung von IgG zur Verbesserung der Antikörperbindung an Polystyrol-Tubes oder -Perlen auszunützen. Die IgG-Lösung wird dabei mit einer sauren, hochmolaren Lösung "verdünnt" und nach kurzer Zeit mit einer zweiten, ebenfalls hochmolaren Lösung wieder "hochgepuffert". Nach diesem Verfahren wird außerdem mit großer Ionenstärke gearbeitet. Ein Nachteil dieses Verfahrens besteht darin, daß ein Teil der Antikörper irreversibel ausfällt und damit zur Bindung an der Oberfläche nicht mehr zur Verfügung steht.

Erfindungsgemäß kann die saure Vorbehandlung der Proteine (bei denen es sich aufgrund der Zweckbestimmung - immunologische Analyse - vorzugsweise um Antikörper handelt) in situ, also während des Aufbringens (Beladens) auf das Faservlies erfolgen, z. B. durch Imprägnieren des Vlieses mit einer sauren wäßrigen Lösung des Proteins; während dieses Schrittes wird das Protein in der sauren Lösung vorbehandelt. Die saure Lösung des Proteins ist vorzugsweise eine Lösung des Proteins in einer geeigneten wäßrigen Pufferlösung mit einem schwach sauren pH-Wert, vorzugsweise mit einem pH-Wert von 3,0 bis 6,0.

Bevorzugt ist jedoch eine getrennte Vorbehandlung des Proteins vor dem Aufbringen auf das Faservlies. Als Antikörper werden dabei vorzugsweise DE-Antikörper (DE-Fraktionen) eingesetzt, wie sie nach Passage über DEAE-Zellulose erhalten werden können. Die saure Vorbehandlung der Proteine kann auf irgendeine geeignete, z. B. aus dem Stand der Technik bekannte Weise erfolgen. Für die getrennte Vorbehandlung haben sich insbesondere die beiden folgenden Verfahren als zweckmäßig erwiesen:

1. Der DE-Antikörper wird mehrfach gegen eine verdünnte Säurelösung dialysiert und die Antikörperlösung wird dann anschließend lyophilisiert. Die Beladung des Faservlieses erfolgt dann mit einer wäßrigen Lösung des erhaltenen Lyophilisats mit einem pH-Wert von 6 bis 7. Als Säure wird z. B. Milchsäure, Salzsäure, Propionsäure, Essigsäure oder Weinsäure in einer geeigneten Konzentration eingesetzt. In einer zweckmäßigen Ausführungsform beträgt z. B. die Konzentration für Salzsäure 1 bis 50 mM, insbesondere 2 bis 10 mM, für die genannten organischen Säuren 1 bis 100 mM, insbesondere 5 bis 30 mM. Der Antikörper (DE-Fraktion) wird z. B. 5 Stunden bis 5 Tage gegen die verdünnte Säurelösung dialysiert. Bevorzugt ist eine Dialysezeit von 12 bis 15 Stunden. Nach der Dialyse wird das Protein zur Lagerung lyophilisiert. Zur Beladung der Vliese mit dem Antikörper wird das Lyophilisat mit Pufferlösung aufgenommen.

2. Der DE-Antikörper (vorzugsweise als Lyophilisat) wird in verdünnter Säurelösung vom pH-Wert 2 bis 4 aufgenommen und diese Lösung zur Beladung eingesetzt. Als Säure eignet sich z. B. Maleinsäure, Phosphorsäure und insbesondere Milchsäure. Zur Herstellung der Lösung eignen sich im Prinzip alle Pufferlösungen mit einem entsprechenden pH-Wert.

Ein Vlies der Dicke 1,5 mm aus 80 Gew.-% Polyester und 20 Gew.-% Sulfitzellstoff bindet nach dem Verfahren 1 ca. 40 μg Antikörper/Vlies der Größe 6 mm x 6 mm (was 2,5 mg Antikörper/g Fasermaterial entspricht); die Bindung nach dem Verfahren 2 liegt in der gleichen Größenordnung. Gegebenenfalls kann eine nach Verfahren 1 mit Antikörper beladene Matrix noch einer nachfolgenden Säurebehandlung

4

unterworfen werden; in der Regel wird die Matrix dadurch in ihrer Funktion aber nicht mehr wesentlich verbessert.

Mit dem erfindungsgemäßen Verfahren können gegenüber dem Stand der Technik wesentliche, nicht vorhersehbare Vorteile erzielt werden, und zwar sowohl hinsichtlich der Durchführbarkeit des Verfahrens als auch hinsichtlich der Reagenzpapiere und der damit durchzuführenden immunologischen Analyse. Solche Vorteile sind insbesondere:

Das Verfahren eignet sich auch sehr gut für große Ansätze; so erlaubt z. B. die Perjodat-Behandlung unter Verwendung eines Baumfärbeapparats und die kontinuierliche Imprägnierung mit anschließender Trocknung des Faservlieses in einer üblichen Imprägnieranlage große Ansätze. Diese Verfahren sind insbesondere geeignet für die kontinuierliche Herstellung flächiger Reagenzpapiere der Größe 30 bis 50 $mm^2$ einem Ansatz von 10 bis 50 $m^2$ bei Verwendung der in der Papierherstellung üblichen technischen Geräte. Hierbei können die zur Bestimmung von niedrigkonzentrierten Analyten erforderlichen engen Qualitätstoleranzen ohne Schwierigkeiten eingehalten werden. Die Beladung der Faservliese durch Imprägnierung mit Proteinlösungen erlaubt außerdem eine genau einstellbare Dosierung und die Aufkonzentrierung der Proteinlösungen in einer Trocknungsstufe hohe Beladungsdichten. Nach dem erfindungsgemäßen Verfahren werden Beladungsdichten im Mikrogramm-Bereich/Test, z. B. 40 µg Antikörper/Test, erhalten, gegenüber ca. 100 bis max. 1000 ng/Test nach dem Stand der Technik, z. B. 100 bis 200 ng Antikörper/Test bei Verwendung eines reinen Polyesterpapieres.

Kurze Kopplungszeiten, die z. B. in einer technischen Anlage nicht mehr als 10 Minuten betragen, tragen ebenfalls zur Wirtschaftlichkeit des Verfahrens bei. Das Verfahren kann ansatzweise oder kontinuierlich durchgeführt werden, wobei bei der kontinuierlichen Durchführung in der Regel ein noch gleichmäßigeres Produkt erhalten wird. Aufgrund des einfachen Verfahrens (z. B. kann die Perjodatoxidation ohne den nachträglichen Reduktionsschritt, wie er nach dem Stand der Technik erforderlich ist, durchgeführt werden) und der kurzen Beladungszeiten kann das Verfahren auch im technischen Maßstab kontinuierlich durchgeführt werden, was für die industrielle Herstellung von Reagenzpapieren von wesentlicher Bedeutung ist. Nach dem erfindungsgemäßen Verfahren ist eine gute Fixierung der Proteine auf dem Trägermaterial überraschenderweise auch ohne zusätzliche Fixierungsschritte möglich; dies war keinesfalls naheliegend: nach dem Stand der Technik schließt sich an die Perjodatbehandlung immer eine Hydrierung an, weil angenommen wird, daß sonst keine ausreichende Stabilität erhalten werden kann; eine solche nachfolgende Hydrierung ist nach dem erfindungsgemäßen Verfahren nicht erforderlich.

Mit dem erfindungsgemäßen Reagenzpapier treten keine Desorptionserscheinungen während der immunologischen Bestimmungen und bei hoher Bindekapazität keine unspezifischen Bindungen mit den markierten Antikörpern auf; aufgrund der hohen Beladungsdichten sind bei den immunologischen Tests im Vergleich zu bekannten Reagenzpapieren wesentlich kürzere Reaktionszeiten möglich. Erfindungsgemäß werden somit Reagenzpapiere bereitgestellt, die neben einer hohen Bindekapazität gleichzeitig eine niedrige unspezifische Fixierung anderer in der Flüssigkeit vorhandener Substanzen aufweisen. Erfindungsgemäß gelingt es also, die Bindekapazität zu steigern, ohne die Fixierung von unspezifisch gebundenen Substanzen in gleicher Weise zu erhöhen; dies ist deshalb überraschend, weil normalerweise eine Steigerung der Bindekapazität auch zu einer Steigerung der unspezifischen Adsorption führt.

Die erfindungsgemäßen Reagenzpapiere zeigen eine hohe Empfindlichkeit bei geringer Streuung und niedrigen Leerwerten; aufgrund der stabilen Bindung des Proteins auf dem Träger können sie z. B. mit Detergentien gewaschen werden, wodurch eine Interferenz mit Plasma und anderen Probebestandteilen gering gehalten werden kann. Aufgrund ihrer Eigenschaften stellen die erfindungsgemäßen Reagenzpapiere somit ein homogenes Reagenz zur immunologischen Analyse dar, das z. B. durch Schneiden in eine geeignete Form und Größe exakt dosierbar ist. Aufgrund ihrer hohen Stabilität eignen sich die erfindungsgemäßen Reagenzpapiere auch sehr gut für die Verwendung und den Vertrieb in Behältern, die alle notwendigen Reagenzien und Reaktionsgefäße für die immunologische Bestimmung enthalten. Sie können auch einfach in Vertiefungen von Mikrotiterplatten oder in Röhrchen eingebracht werden, und eignen sich aufgrund ihrer Eigenschaften auch gut für die Verwendung als Teststreifen.

Die nachfolgenden Beispiele erläutern die Erfindung näher. Wenn nicht anders angegeben, beziehen sich Temperaturangaben auf die Celsius-Skala, und Prozent- und Mengenangaben auf Gewichtsprozent und Gewichtsteile.

**Beispiele**

**Beispiel 1**

Dieses Beispiel beschreibt die Aufreinigung von Antikörpern zur DE-Fraktion.

Schaf-anti-Maus-Fcγ-Antiserum wird auf 1,8 Mol mit Natriumsulfat versetzt. Das Präzipitat wird in PBS (phosphate buffered saline) pH = 7,0 aufgenommen und die so erhaltene Lösung einer Passage über DEAE-Zellulose unterworfen. Die IgG enthaltende Fraktion wird bis zur Verwendung eingefroren (-20 °C).

**Beispiel 2**

Dieses Beispiel beschreibt die Aktivierung und Beladung eines Vliesmaterials, wobei ein möglichst homogenes Vliesmaterial, d. h. mit einer homogenen Faserverteilung, und mit hoher Flächengewichts- und Dickenkonstanz

$$\text{(Flächengewichts-VK} \stackrel{\scriptscriptstyle\wedge}{=} 5 \text{ \%, Dicken-VK} \stackrel{\scriptscriptstyle\wedge}{=} 5 \text{ \% (VK = Variations-Koeffizient))}$$

eingesetzt wird.

Das Vlies besteht aus 90 % Polyester, 10 % Cellulosefasern und 1 % Etadurin® (bezogen auf die Menge an Cellulosefasern) als Naßfestmittel.

a) Aktivierung:

Die Aktivierung des Vliesmaterials (10 bis 50 m²) erfolgt in einem Baumfärbeapparat. Als Oxidationsmedium dient 10 mmolare NaJO₄-Lösung. Perjodatmenge: 2 mM/g Vlies, Oxidationszeit: 2 Stunden. Nach dieser Behandlung wird das Vliesmaterial im Färbebaum perjodatfrei gewaschen und in einem Schwebetrockner getrocknet.

$$\text{Restfeuchte} \stackrel{\scriptscriptstyle\wedge}{=} 5 \text{ \%.}$$

b) Beladung mit Protein (Antikörper):

Das nach a) behandelte Vliesmaterial wird auf einer üblichen Imprägnieranlage mit einer Proteinlösung getränkt. Zusammensetzung der Proteinlösung:

Puffersalze, 1 bis 20 mg Antikörper (Schaf-anti-Maus-Fcγ), pH-Wert der Lösung: 3,0 bis 6,0. Vorschubgeschwindigkeit der Imprägnieranlage: 0,1 m/min. Das imprägnierte Vliesmaterial wird dann auf der Imprägnieranlage on-line mittels eines Schwebetrockners getrocknet.

$$\text{Restfeuchte} < 5 \text{ \%.}$$

Die Reaktion Protein-Vlies erfolgt in der Naßstrecke (ca. 5 bis 8 m). Reaktionszeit < 5 min.

c) Nachbehandlung:

Das nach b) beladene Vlies wird in einem Färbebaum mit Pufferlösungen (pH = 6 bis 8) gewaschen, um ungebundenes Protein zu entfernen. Anschließend wird das Vlies auf einer üblichen Imprägnieranlage mittels eines Schwebetrockners getrocknet.

$$\text{Restfeuchte} < 5 \text{ \%.}$$

**Beispiel 3**

Dieses Beispiel beschreibt die Verwendung eines erfindungsgemäßen Reagenzpapieres als Immunadsorber zur Bestimmung von Isoamylase.

Ein erfindungsgemäßes, z. B. in den vorstehenden Beispielen beschriebenes Antikörpervlies wird mit

6

einem monoklonalen Antikörper (Maus), vgl. DE 33 42 736, gegen h-Speichelamylase getränkt (c ∿ 50 µg/ml; spezifische Flüssigkeitsaufnahme des Vlieses ca. 700 ml/m²) und anschließend wie in Beispiel 2 beschrieben getrocknet.

Aus dem so beladenen Vlies werden kleine Stücke ausgeschnitten oder ausgestanzt. Zur Entfernung von etwa 1000 U/l Speichelamylase aus 500 µl h-Serum benötigt man ca. 1 cm² Papier. Das Serum wird 30 Minuten bei Raumtemperatur mit dem Immunosorbens-Papier geschüttelt. Die Restaktivität an h-Speichelamylase in dem so behandelten Serum beträgt 1 bis 5 %, die Restaktivität an h-Pankreasamylase ist größer als 90 %.

## Beispiel 4

Bestimmung von Thyreotropin (TSH)

Es werden drei Antikörper (Rezeptoren) verwendet.

Rezeptor 1 und 3 stammen von derselben Tierspezies (Maus) und sind gegen TSH gerichtet. Rezeptor 3 ist ein markiertes Fab-Fragment. Rezeptor 1 ist ein monoklonaler Antikörper, das Fab-Fragment in Rezeptor 3 stammt ebenfalls aus einem monoklonalen Antikörper, der gegen eine andere Determinante des TSH gerichtet ist als der monoklonale Antikörper von Rezeptor 1. Rezeptor 2 stammt aus Schaf und ist gegen den Fc-Teil der monoklonalen Mausantikörper gerichtet. Die Entwicklung von monoklonalen Antikörpern erfolgte nach der Methode von Köhler und Milstein, Eur. J. Immunol. 6, 292 (1976).

Reagenzien:

1. Inkubationspuffer (IP):

100 mM Natrium-Phosphat-Puffer, pH 7,4.

2. Rezeptor 1:

Maus-anti-TSH-Antiserum (Rezeptor 1):

Die monoklonale Anti-TSH-Antikörper enthaltende Ascitesflüssigkeit aus Mäusen wird ad 1,8 M mit $NH_4SO_4$ versetzt. Das Präzipitat wird in einem Puffer aus 15 mM Natriumphosphat, pH 7,0 und 50 mM NaCl aufgenommen, und die so erhaltene Lösung wird einer Passage über DEAE-Cellulose unterworfen.

3. Rezeptor 3:

Anti-TSH-Antikörper (monoklonal), der eine andere antigene Determinante als Rezeptor 1 erkennt; Peroxidase-Konjugat (Rezeptor 3): Maus-anti-TSH-Antiserum wird wie Rezeptor 1 aufgereinigt. Die anschließende Gewinnung von Fab aus dem kompletten Antikörpermolekül erfolgt nach der Methode von R. R. Porter, Biochem. J. 73 (1959), 119. Die Kopplung mit Meerrettich-Peroxidase erfolgt nach der Methode von Nakane (M. B. Wilson, P. K. Nakane "Recent Developments in the Periodate Method of Conjugating Horseradish Peroxidase to Antibodies", 1978, Elsevier, North Holland Biomedical Press. S. 215-224, in "Immunofluorescence and Related Staining Techniques").

4. Rezeptor 2:

Fixierung von Schaf-anti-Maus-Fcγ-Antikörper (Rezeptor 2 Adsorber):

Schaf-anti-Maus-Fcγ-Antiserum wird ad 1,8 M mit $NH_4SO_4$ versetzt. Das Präzipitat wird in einem Puffer aus 15 mM Natriumphosphat, pH 7,0 und 50 mM NaCl aufgenommen und die so erhaltene Lösung einer Passage über DEAE-Cellulose unterworfen. Die IgG enthaltende Fraktion (Rezeptor 2) wird nach Beispiel 2 weiterbehandelt.

5. Indikatorreagenz:

1,8 mM ABTS (2,2'-Azino-di-(3-ethylbenzthiazolinsulfonat-(6))),3,3 mM Natrium-Perborat in 100 mM Phosphat-Citrat-Puffer, pH 4,4.

Durchführung:

a) Doppelantikörper-Sandwich

50 ng Rezeptor 1 und 170 mU Rezeptor 3 werden in Inkubationspuffer gelöst zusammen auf ein käufliches Zellulosevlies von der Größe 6 mm x 6 mm, Dicke 1,0 mm, aufgetropft und bei Raumtemperatur getrocknet. Dieses Reagenz enthaltende Papiervlies wird Reagenzträger 1 genannt.

Auf Reagenzträger 1 werden 40 µl der zu bestimmenden Probe bzw. bekannte Mengen Antigen enthaltenden Standardlösung pipettiert, anschließend wird das Vlies in einer Eppendorf-Zentrifuge sofort abzentrifugiert, wobei Reagenzträger 1 auf einem Eppendorf-Hütchen placiert wird und die eluierte Flüssigkeit im Hütchen während der Zentrifugation auf den Rezeptor 2 trifft und mit diesem reagiert. Von Rezeptor 2 wird ein erfindungsgemäßes Vlies der Papiermischung Polyester 80 % und Zellulose 20 % und der Größe 6 x 6 mm, Dicke 0,7 mm, pro Test eingesetzt.

Auf einer Schüttelapparatur wird das Reaktionsgemisch 15 Minuten bei 37° C inkubiert, anschließend wird dreimal mit je 1 ml IP gewaschen und schließlich werden 200 µl Indikatorreagenz zugegeben. Mit zwei verschiedenen TSH-Proben wurden folgende Extinktionen ermittelt:

| TSH ($\mu U/ml$) | $E_{405\ nm/cm}$* |
|---|---|
| 0 | 0,74 |
| 50 | 7,54 |

\* Die Extinktionswerte wurden bei 0,2 cm Schichtdicke ermittelt und auf 1 cm Schichtdicke umgerechnet.

b) Einfacher Sandwich

Die Durchführung erfolgt mit den gleichen Reagenzien und in gleicher Weise wie unter a) beschrieben, aber mit folgenden Änderungen:

1. 1 Vlies (Polyester 80 %, Zellulose 20 %) mit Rezeptor 2 wird 1 Stunde mit 20 µg Rezeptor 1 in 0,2 ml vorinkubiert. Anschließend wird der Überstand abgesaugt und das Vlies dreimal mit je 1 ml IP gewaschen.

2. Derart mit Rezeptor 1 vorbehandelter Adsorber-Rezeptor 2 wird 4 Stunden mit Probe und Rezeptor 3 auf einer Schüttelapparatur bei 37° C inkubiert; die Zugabe von löslichem Rezeptor 1 entfällt in dieser Variante.

Anschließend wird wie üblich der Überstand abgesaugt, das Vlies (Rezeptor 2) gewaschen und die fixierte Enzymaktivität mit Indikatorreagenz (200 µl) nachgewiesen.

Mit 2 verschiedenen TSH-Proben wurden folgende Extinktionswerte ermittelt:

| TSH<br>($\mu$U/ml) | $E_{405}$ nm/cm* |
|---|---|
| 0 | 0,20 |
| 50 | 2,87 |

\* Die Extinktionswerte wurden bei 0,2 cm Schichtdicke ermittelt und auf 1 cm Schichtdicke umgerechnet.

**Beispiel 5** (Vergleichsbeispiel)

Es wird die Testperformance in Abhängigkeit von der Papierrezeptur verglichen, und zwar ein erfindungsgemäß hergestelltes Reagenzpapier (Papier 3) mit einem Papier aus 100 % Zellulose (Papier 1) und einem Papier aus 100 % Polyester (Papier 2).

Alle drei Reagenzpapiere wurden in einem Enzymimmunoassay zur Bestimmung von carcinoembryonalem Antigen (CEA) verglichen. Das Verfahren zur Kopplung von Antikörpern (Schaf-anti-Maus-Fc$\gamma$) war in allen drei Fällen identisch; es wurden 5 mg Antikörper pro g Fasermaterial verwendet. Die Proteinbeladung und übrigen Verfahrensmaßnahmen erfolgten wie in Beispiel 2 beschrieben.

Es wurde nach dem Sandwich-Prinzip in Analogie zu Beispiel 4 gearbeitet. In Abänderung zu den Angaben dieses Beispiels wurden monoklonale Antikörper (gegen carcinoembrionales Antigen gerichtet) verwendet. In der nachfolgenden Tabelle sind die Daten für die unspezifische Bindung (Leerwert) und für den dynamischen Meßbereich (darunter ist die Differenz zwischen dem Signal für den höchsten Standard und dem Nullstandard zu verstehen) für die drei Reagenzpapiere angegeben.

| Papier | Leerwert | Dynamischer Meßbereich | Untere Nach-weisgrenze |
|---|---|---|---|
| 1 | 2000 mE | 1000 mE | 30 ng/ml |
| 2 | 100 mE | 300 mE | 5 ng/ml |
| 3 | 600 mE | 5800 mE | 1,55 ng/ml |

Die Extinktionen wurden bei $\lambda$ = 578 nm unter Verwendung einer 3 mm-Küvette gemessen und auf eine Schichtdicke von d = 1 cm umgerechnet.

Ein Vergleich der in Tabelle 1 aufgezeigten Werte zeigt deutlich, daß mit dem erfindungsgemäß hergestellten Reagenzpapier (Papier 3) wesentlich bessere Ergebnisse als mit den nicht erfindungsgemäßen Reagenzpapieren (Papier 1 und Papier 2) erhalten werden können.

**Ansprüche**

1. Reagenzpapier für die immunologische Analyse, **gekennzeichnet durch** ein Faservlies aus einer Mischung eines Zellulose-Bestandteils und einer Synthesefaser worin das Gewichtsverhältnis Zellulose-Bestandteil /synthesefaser 1 bis 90/99 bis 10 beträgt, wobei das Faservlies mit Perjodat aktiviert und mit einem sauer vorbehandelten Protein beladen ist.

2. Verfahren zur Herstellung eines Reagenzpapieres gemäß Anspruch 1, **dadurch gekennzeichnet,** daß man ein Faservlies aus einer Mischung von einem Zellulose-Bestandteil und einer Synthesefaser, worin das Gewichtsverhältnis von Zellulose-Bestandteil/Synthesefaser 1 bis 90/99 bis 10 beträgt, durch Behandlung mit Perjodat aktiviert, das so aktivierte Faservlies mit einem sauer behandelten Protein belädt und ungebundenes Protein entfernt.

3. Verfahren nach Anspruch 2, **dadurch gekennzeichnet,** daß das Gewichtsverhältnis Zellulose-Bestandteil/Synthesefaser 10 bis 80/20 bis 90, insbesondere 20 bis 40/60 bis 80 beträgt.

4. Verfahren nach Anspruch 2 oder 3,
**dadurch gekennzeichnet,**
daß man als Zellulosebestandteil Zellulose, Sulfitzellstoff, Sulfatzellstoff und/oder Linters verwendet.

5. Verfahren nach einem der Ansprüche 2 bis 4,
**dadurch gekennzeichnet,**
daß man als Synthesefasern Polyamid, Polyacrylnitril, Zellwolle, Glasfasern und/oder insbesondere Polyester verwendet.

6. Verfahren nach einem der Ansprüche 2 bis 5,
**dadurch gekennzeichnet,**
daß man zusätzlich zu den Synthesefasern Bindefasern oder/und Naßfestmittel in einer Menge von 0,1 bis 30 Gew.-%, bezogen auf die Menge an Zellulose-Bestandteil verwendet.

7. Verfahren nach Anspruch 6 , **dadurch gekennzeichnet,** daß man als Bindefasern Polyvinylalkohol, Polyurethan, Polystyrol und/oder Polyvinylchlorid verwendet.

8. Verfahren nach Anspruch 6, **dadurch gekennzeichnet,** daß man als Naßfestmittel Polyamid-Epichlorhydrine, HarnstoffFormaldehyde und/oder Melamin-Formaldehyde verwendet.

9. Verfahren nach einem der Ansprüche 2 bis 8 ,
**dadurch gekennzeichnet,**
daß man die Aktivierung der Fasern mit Perjodat vor der Bildung des Vlieses vornimmt.

10. Verfahren nach Anspruch 9, **dadurch gekennzeichnet,** daß man nur den Zellulose-Bestandteil einer Behandlung mit Perjodat unterwirft.

11. Verfahren nach einem der Ansprüche 2 bis 10,
**dadurch gekennzeichnet,**
daß man die saure Vorbehandlung des Proteins in situ durchführt.

12. Verfahren nach einem der Ansprüche 2 bis 11,
**dadurch gekennzeichnet,**
daß man als Protein einen Antikörper einsetzt.

13. Verfahren nach Anspruch 12, **dadurch gekennzeichnet,** daß man den Antikörper gegen eine Säurelösung dialysiert, die erhaltene Antikörper-Lösung dann lyophilisiert, und das Faservlies mit dem so behandelten Antikörper in wäßriger Lösung bei pH = 6 bis 7 imprägniert.

14. Verfahren nach Anspruch 13, **dadurch gekennzeichnet,** daß man als Säure Milchsäure, Maleinsäure, Salzsäure, Propionsäure, Essigsäure oder Weinsäure einsetzt.

15. Verfahren nach Anspruch 13, **dadurch gekennzeichnet,** daß man zur Beladung des Faservlieses den Antikörper in einer Säurelösung vom pH = 2 bis 4 aufnimmt und das Faservlies mit dieser Lösung imprägniert.

16. Verfahren nach einem der Ansprüche 2 bis 15,
**dadurch gekennzeichnet,**
daß man ungebundenes Protein durch Waschen des beladenen Vlieses mit wäßrigen Pufferlösungen vom pH = 6 bis 8 entfernt.

17. Verwendung eines Reagenzpapiers nach Anspruch 1 als Reagensträger für die heterogene immunologische Analyse.

18. Verwendung nach Anspruch 17, **dadurch gekennzeichnet,** daß das Gewichtsverhältnis Zellulose-Bestandteil/Synthesefaser 10 bis 80/20 bis 90, insbesondere 20 bis 40/60 bis 80 beträgt.

19. Verwendung nach Anspruch 17 oder 18,
**dadurch gekennzeichnet,**
daß das Faservlies als Zellulosebestandteil Zellulose, Sulfitzellstoff, Sulfatzellstoff und/oder Linters enthält.

20. Verwendung nach einem der Ansprüche 17 bis 19,
**dadurch gekennzeichnet,**
daß das Faservlies als Synthesefasern Polyamid, Zellwolle, Polyacrylnitril, Glasfasern und/oder insbesondere Polyester enthält.

21. Verwendung nach einem der Ansprüche 17 bis 19,
**dadurch gekennzeichnet,**
daß das Faservlies zusätzlich zu den Synthesefasern Bindefasern oder Naßfestmittel in einer Menge von 0,1 bis 30 Gew.-%, bezogen auf die Menge an Zellulose-Bestandteil, enthält.

22. Verwendung nach Anspruch 21 , **dadurch gekennzeichnet,** daß das Faservlies als Bindefasern Polyvinylalkohol, Polyurethan, Polystyrol und/oder Polyvinylchlorid enthält.

23. Verwendung nach Anspruch 21 , **dadurch gekennzeichnet,** daß das Faservlies als Naßfestmittel Polyamid-Epichlorhydrine, Harnstoff-Formaldehyde und/oder Melamin-Formaldehyde enthält.

## Claims

1. Reagent paper for immunological analysis, characterised by a fibre fleece of a mixture of a cellulose component and of a synthetic fibre, wherein the weight ratio of cellulose component/synthetic fibre amounts to 1 to 90/99 to 10, whereby the fibre fleece is activated with periodate and is loaded with an acid pre-treated protein.

2. Process for the production of a reagent paper according to claim 1, characterised in that one activates a fibre fleece of a mixture of a cellulose component and of a synthetic fibre, wherein the weight ratio of cellulose component/synthetic fibre amounts to 1 to 90/99 to 10, by treatment with periodate, loads the so activated fibre fleece with an acid-treated protein and removes non-bound protein.

3. Process according to claim 2, characterised in that the weight ratio of cellulose component/synthetic fibre amounts to 10 to 80/20 to 90, especially to 20 to 40/60 to 80.

4. Process according to claim 2 or 3, characterised in that, as cellulose component, one uses cellulose, sulphite cellulose, sulphate cellulose and/or linters.

5. Process according to one of claims 2 to 4, characterised in that, as synthetic fibres, one uses polyamide, polyacrylonitrile, regenerated cellulose, glass fibres and polyester.

6. Process according to one of claims 2 to 5, characterised in that, in addition to the synthetic fibres, one

11

uses binding fibres and/or wet strength agents in an amount of 0.1 to 30 wt.%, referred to the amount of cellulose component.

7. Process according to claim 6, characterised in that, as binding fibres, one uses polyvinyl alcohol, polyurethane, polystyrene and/or polyvinyl chloride.

8. Process according to claim 6, characterised in that, as wet strength agents, one uses polyamideepichlorohydrins, urea-formaldehydes and/or melamineformaldehydes.

9. Process according to one of claims 2 to 8, characterised in that one carries out the activation of the fibres with periodate before the formation of the fleece.

10. Process according to claim 9, characterised in that one only subjects the cellulose component to a treatment with periodate.

11. Process according to one of claims 2 to 10, characterised in that one carries out the acid pretreatment of the protein in situ.

12. Process according to one of claims 2 to 11, characterised in that one uses an antibody as protein.

13. Process according to claim 12, characterised in that one dialyses the antibody against an acid solution, then lyophilises the antibody solution obtained and impregnates the fibre fleece with the so treated antibody in aqueous solution at pH = 6 to 7.

14. Process according to claim 13, characterised in that one uses lactic acid, maleic acid, hydrochloric acid, propionic acid, acetic acid or tartaric acid as acid.

15. Process according to claim 13, characterised in that, for the loading of the fibre fleece, one takes up the antibody in an acid solution of the pH = 2 to 4 and impregnates the fibre fleece with this solution.

16. Process according to one of claims 2 to 15, characterised in that one removes non-bound protein by washing of the loaded fleece with aqueous buffer solutions of pH = 6 to 8.

17. Use of a reagent paper according to claim 1 as reagent carrier for heterogeneous immunological analysis.

18. Use according to claim 17, characterised in that the weight ratio of cellulose component/synthetic fibre amounts to 10 to 80/20 to 90, especially to 20 to 40/60 to 80.

19. Use according to claim 17 or 18, characterised in that, as cellulose component, the fibre fleece contains cellulose, sulphite cellulose, sulphate cellulose and/or linters.

20. Use according to one of claims 17 to 19, characterised in that, as synthetic fibres, the fibre fleece contains polyamide, regenerated cellulose, polyacrylonitrile, glass fibres and/or polyester.

21. Use according to one of claims 17 to 19, characterised in that, in addition to the synthetic fibres, the fibre fleece contains binding fibres or wet strength agents in an amount of 0.1 to 30 wt.%, referred to the amount of cellulose component.

22. Use according to claim 21, characterised in that, as binding fibres, the fibre fleece contains polyvinyl alcohol, polyurethane, polystyrene and/or polyvinyl chloride.

23. Use according to claim 21, characterised in that, as wet strength agent, the fibre fleece contains polyamide-epichlorohydrins, urea-formaldehydes and/or melamine-formaldehydes.

**Revendications**

1. Papier réactif pour l'analyse immunologique, caractérisé par un matelas de fibres ou non-tissé constitué d'un mélange d'un constituant cellulosique et d'une fibre de synthèse, dans lequel le rapport en poids du constituant cellulosique à la fibre de synthèse est de 1 jusqu'à 90/99 jusqu'à 10, le matelas de fibres ou non-tissé étant activé au periodate et chargé avec une protéine prétraitée à l'acide.

2. Procédé pour la fabrication d'un papier réactif selon la revendication 1, caractérisé en ce qu'on active un matelas de fibres ou non-tissé constitué d'un mélange d'un constituant cellulosique et d'une fibre de synthèse, dans lequel le rapport en poids du constituant cellulosique à la fibre de synthèse est de 1 Jusqu'à 90/99 Jusqu'à 10, par traitement au periodate, on charge le matelas de fibres ou non-tissé ainsi activé avec une protéine traitée à l'acide et on élimine la protéine non liée.

3. Procédé selon la revendication 2, caractérisé en ce que le rapport en poids du constituant cellulosique à la fibre de synthèse est de 10 jusqu'à 80/20 jusqu'à 90, en particulier de 20 jusqu'à 40/60 jusqu'à 80.

4. Procédé selon la revendication 2 ou 3, caractérisé en ce que l'on utilise comme constituant cellulosique de la cellulose, de la pâte au sulfite, de la pâte au sulfate et/ou des linters.

5. Procédé selon l'une des revendications 2 à 4, caractérisé en ce que l'on utilise comme fibres de synthèse du polyamide, du polyacrylonitrile, de la fibranne, des fibres de verre et ou en particulier du polyester.

6. Procédé selon l'une des revendications 2 à 5, caractérisé en ce qu'on utilise, outre les fibres de synthèse, des fibres de liaison ou/et des agents résistants à l'état humide en une quantité de 0,1 à 30% en poids par rapport à la quantité de constituant cellulosique.

7. Procédé selon la revendication 6, caractérisé en ce que l'on utilise comme fibres de liaison de l'alcool polyvinylique, du polyuréthane, du polystyrène et/ou du polychlorure de vinyle

8. Procédé selon la revendication 6, caractérisé en ce que l'on utilise comme agents résistants à l'état humide des polyamide-épichlorhydrines,des urée-formaldéhydes et/ou des mélamine-formaldéhydes.

9. Procédé selon l'une des revendications 2 à 8, caractérisé en ce qu'on effectue l'activation des fibres par le periodate avant la formation du non-tissé.

10. Procédé selon la revendication 9, caractérisé en ce que l'on soumet seulement le constituant cellulosique à un traitement au periodate.

11. Procédé selon l'une des revendications 2 à 10, caractérisé en ce qu'on effectue le prétraitement acide de la protéine in situ.

12. Procédé selon l'une des revendications 2 à 11, caractérisé en ce qu'on utilise comme protéine un anticorps.

13. Procédé selon la revendication 12, caractérisé en ce qu'on dialyse l'anticorps contre une solution d'acide, on lyophilise ensuite la solution d'anticorps obtenue, et on imprégne le non-tissé avec l'anticoprs ainsi traité en solution aqueuse à pH = 6 jusqu'à 7.

14. Procédé selon la revendication 13, caractérisé en ce qu'on utilise comme acide l'acide lactique, l'acide maléique, l'acide chlorhydrique, l'acide propionique, l'acide acétique ou l'acide tartrique.

15. Procédé selon la revendication 13, caractérisé en ce qu'on reprend l'anticorps dans une solution acide de pH = 2 à 4 pour charger le non-tissé et en ce qu'on imprégne le non-tissé avec cette solution.

16. Procédé selon l'une des revendications 2 à 15, caractérisé en ce que l'on élimine la protéine non liée par lavage du non-tissé chargé avec des solutions tampons aqueuses de pH = 6 à 8.

17. Utilisation d'un papier réactif selon la revendication 1 comme support de réactif pour l'analyse immunologique hétérogène.

18. Utilisation selon la revendication 17, caractérisée en ce que le rapport en poids du constituant cellulosique à la fibre de synthèse est de 10 jusqu'à 80/20 jusqu 'à 90, en particulier de 20 jusqu 'à 40/60 jusqu'à 80.

19. Utilisation selon la revendication 17 ou 18, caractérisée en ce que le non-tissé contient comme constituant cellulosique de la cellulose, de la pâte au sulfite, de la pâte au sulfate et/ou des linters.

20. Utilisation selon l'une des revendications 17 à 19, caractérisée en ce que le non-tissé contient comme fibres de synthèse du polyamide, de la fibranne, du polyacrylonitrile, des fibres de verre et/ou en particulier du polyester.

21. Utilisation selon l'une des revendications 17 à 19, caractérisée en ce que le non-tissé contient, outre les fibres de synthèse, des fibres de liaison ou des agents résistants à l'état humide, en une quantité de 0,1 à 30% en poids, par rapport à la quantité de constituant cellulosique.

22. Utilisation selon la revendication 21, caractérisée en ce que le non-tissé contient comme fibres de liaison de l'alcool polyvinylique, du polyuréthane, du polystyrène et/ou du polychlorure de vinyle.

23. Utilisation selon la revendication 21, caractérisée en ce que le non-tissé contient comme agents résistants à l'état humide, des polyamide-épichlorhydrines, des urée-formaldéhydes et/ou des mélamine-formaldéhydes.